(19)

**Europäisches Patentamt**
**European Patent Office**
**Office européen des brevets**

(11) **EP 4 603 856 A1**

(12) **EUROPEAN PATENT APPLICATION**

(43) Date of publication:
20.08.2025 Bulletin 2025/34

(21) Application number: 25157179.0

(22) Date of filing: 11.02.2025

(51) International Patent Classification (IPC):
*G01R 33/28* (2006.01)     *A61B 5/11* (2006.01)
*A61B 5/00* (2006.01)      *G01R 33/565* (2006.01)

(52) Cooperative Patent Classification (CPC):
**G01R 33/56509; G01R 33/288**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.02.2024 JP 2024020707**

(71) Applicant: **FUJI-FILM Corporation**
**Minato-ku**
**Tokyo 106-8620 (JP)**

(72) Inventors:
• **KANEKO, Yukio**
  **Tokyo (JP)**
• **SHIRAI, Toru**
  **Tokyo (JP)**
• **IKEGAWA, Ayaka**
  **Tokyo (JP)**

(74) Representative: **Dehns Germany Partnerschaft mbB**
**Theresienstraße 6-8**
**80333 München (DE)**

(54) **NUCLEAR MAGNETIC RESONANCE IMAGING APPARATUS AND BODY MOVEMENT INFORMATION PROCESSING METHOD**

(57)     Provided are a nuclear magnetic resonance imaging apparatus and a body movement information processing method capable of detecting and classifying a body movement of a subject. A nuclear magnetic resonance imaging apparatus includes: an imaging unit (20) that measures a nuclear magnetic resonance signal generated by a subject and that acquires an image of the subject; and a body movement information processing unit (10) that includes a processor and that processes movement information of the subject disposed on an imaging apparatus, in which the body movement information processing unit includes a body movement information calculation unit (13) and a body movement information classification unit (15), and the processor receives a signal from a measurement device (30) that measures the movement information of the subject and calculates body movement information from the signal, and classifies the body movement information.

**FIG. 1**

EP 4 603 856 A1

**Description**

**BACKGROUND OF THE INVENTION**

1. Field of the Invention

The present invention relates to an apparatus that processes information regarding a movement of a subject under an examination.

2. Description of the Related Art

In an examination using a medical image capturing apparatus such as a magnetic resonance imaging apparatus (hereinafter, referred to as an MRI device), the examination is performed in a state in which a subject (patient) is laid on a table or the like or is loosely fixed. In a case in which the subject moves during the examination, an artifact is generated in an image obtained by the medical image capturing apparatus, which hinders diagnosis. In addition, in a case in which the subject makes a large and sudden movement, the subject moves significantly from an examination position, or the subject falls off from a bed, the examination has to be interrupted. However, during the examination, a technician or a doctor (referred to as a user) often focuses his or her attention on a monitor displaying the image and may not even be aware of such incidents.

In the related art, as a technology related to the medical image capturing apparatus, a technology for reducing an influence of a movement of a subject under an examination on a diagnosis image has been developed and proposed.

For example, JP2006-346235A proposes an MRI device in which a body movement is detected by a camera attached in or near an examination space and scanning is stopped or redone in a case in which the body movement is detected.

**SUMMARY OF THE INVENTION**

Incidentally, the body movement includes a large body movement related to safety and a small body movement related to image quality. For example, in a case in which the body movement is a large body movement related to safety, the user needs to go to the subject and provide immediate support. On the other hand, in a case in which the body movement is a small body movement related to image quality, the user can continue imaging. That is, even in a case in which the body movement occurs, the measures that can be taken by the user vary depending on a type of the body movement.

For example, in a case in which the body movement is a large body movement related to safety, the user can immediately support the subject in a case in which the user can be notified of the body movement. In a case in which the body movement is a small body movement related to image quality, an artifact may occur in the image in a case in which the user continues the imaging, but in a case in which the image can be corrected using body movement information, the user does not need to perform re-imaging.

JP2006-346235A does not disclose a method of classifying the type of the body movement. In a case in which it is desired to notify the user of a large body movement related to safety, and such a movement is detected as a small body movement, the notification may be missed, which may be a problem from the viewpoint of safety. In addition, in a case in which a small body movement related to image quality to the extent that the user can continue the examination occurs, and such a movement is notified of the user as a large body movement, the technician's workflow may be hindered.

Even though the user applies body movement correction in a case in which a large body movement is detected that makes it impossible in principle to correct an image artifact, the task of outputting a corrected image itself is a waste, which may hinder the user's workflow.

As described above, it is important to appropriately classify the body movement depending on the use application; however, it is currently difficult to appropriately classify the body movement of the subject.

The present invention has been made in view of such circumstances, and an object of the present invention is to provide a nuclear magnetic resonance imaging apparatus and a body movement information processing method capable of classifying a body movement of a subject.

A first aspect provides a nuclear magnetic resonance imaging apparatus comprising: an imaging unit that measures a nuclear magnetic resonance signal generated by a subject and that acquires an image of the subject; and a body movement information processing unit that includes a processor and that processes movement information of the subject disposed on an imaging apparatus, in which the body movement information processing unit includes a body movement information calculation unit and a body movement information classification unit, and the processor receives a signal from a measurement device that measures the movement information of the subject and calculates body movement information from the signal, and classifies the body movement information.

A second aspect provides the nuclear magnetic resonance imaging apparatus according to the first aspect, in which the processor classifies the body movement information based on at least two or more types of indicators.

A third aspect provides the nuclear magnetic resonance imaging apparatus according to the second aspect, in which the at least two or more types of indicators include a magnitude of a body movement and a duration of the body movement.

A fourth aspect provides the nuclear magnetic resonance imaging apparatus according to the third aspect, in which the at least two or more types of indicators include the magnitude of the body movement, the duration of the body movement, and an envelope curve.

A fifth aspect provides the nuclear magnetic resonance imaging apparatus according to the third or fourth aspect, in which the at least two or more types of indicators further include a spatial domain in which the body movement occurs.

A sixth aspect provides the nuclear magnetic resonance imaging apparatus according to any one of the first to fifth aspects, in which the processor selects a partial region of the subject or a characteristic movement of the subject in a case of calculating the body movement information.

A seventh aspect provides the nuclear magnetic resonance imaging apparatus according to any one of the first to sixth aspects, in which the measurement device is a camera, and the signal is an image captured by the camera, and the processor calculates the body movement information from a temporal change in the image.

An eighth aspect provides the nuclear magnetic resonance imaging apparatus according to any one of the first to sixth aspects, in which the measurement device is a camera, and the signal is an image captured by the camera, and the processor calculates the body movement information from a temporal change in a correlation coefficient of the image.

A ninth aspect provides the nuclear magnetic resonance imaging apparatus according to any one of the first to sixth aspects, in which the measurement device is a stereo camera, and the signal is a stereo image captured by the stereo camera, and the processor calculates the body movement information including three-dimensional information from the stereo image.

A tenth aspect provides the nuclear magnetic resonance imaging apparatus according to any one of the second to fifth aspects, in which the processor sets a threshold value for each of the at least two or more types of indicators, and classifies the body movement information based on the threshold value.

An eleventh aspect provides the nuclear magnetic resonance imaging apparatus according to the tenth aspect, in which the threshold value is a preset value.

A twelfth aspect provides the nuclear magnetic resonance imaging apparatus according to the tenth aspect, in which the threshold value is a value decided by machine learning using previously collected body movement information as correct answer data.

A thirteenth aspect provides the nuclear magnetic resonance imaging apparatus according to the twelfth aspect, in which an algorithm used for the machine learning includes a support vector machine or a decision tree.

A fourteenth aspect provides the nuclear magnetic resonance imaging apparatus according to the twelfth or thirteenth aspect, in which the correct answer data includes augmented correct answer data generated by data augmentation.

A fifteenth aspect provides the nuclear magnetic resonance imaging apparatus according to any one of the first to fourteenth aspects, in which the processor applies a different calculation expression depending on a movement of the subject in a case of calculating the body movement information.

A sixteenth aspect provides the nuclear magnetic resonance imaging apparatus according to any one of the first to fifteenth aspects, in which the processor outputs a classification result from the body movement information processing unit and/or outputs an alert based on the classification result.

A seventeenth aspect provides the nuclear magnetic resonance imaging apparatus according to any one of the first to sixteenth aspects, in which the processor decides whether or not to apply a body movement correction function based on a classification result from the body movement information processing unit.

An eighteenth aspect provides the nuclear magnetic resonance imaging apparatus according to any one of the first to seventeenth aspects, in which the processor applies a body movement correction function based on a classification result from the body movement information processing unit, and outputs a body movement-corrected image based on the body movement information.

A nineteenth aspect provides the nuclear magnetic resonance imaging apparatus according to any one of the first to eighteenth aspects, in which the processor decides a plurality of regions for measuring the movement information of the subject.

A twentieth aspect provides a body movement information processing method of processing movement information of a subject disposed on an imaging apparatus via a body movement information processing device including a processor, the body movement information processing method comprising: causing the processor to receive a signal from a measurement device that measures the movement information of the subject, calculate body movement information from the signal, and classify the body movement information.

According to the present invention, it is possible to classify the body movement information of the subject.

**BRIEF DESCRIPTION OF THE DRAWINGS**

Fig. 1 is a diagram showing an outline of a medical image capturing apparatus.
Fig. 2 is a diagram showing a relationship between a camera and an MRI device.
Fig. 3 is a diagram showing a flow of processing of a body movement information processing device.
Fig. 4 is a diagram showing an example of a medical image capturing apparatus for deciding a threshold value for an indicator.
Figs. 5A and 5B are diagrams for describing processing of calculating body movement information and deciding a threshold value.

Fig. 6 is a diagram for describing a method of extracting feature points from the body movement information extracted in Figs. 5A and 5B.

Fig. 7 is a graph in which feature points of the body movement information extracted based on Figs. 5A and 5B and Fig. 6 are plotted.

Figs. 8A to 8C are diagrams for describing variations in setting a threshold value for an indicator.

Fig. 9 is a diagram for describing a variation of a method of calculating the body movement.

Fig. 10 is a diagram showing an example of a body movement calculated based on a movement vector.

Figs. 11A and 11B are diagrams for describing a method of calculating body movement information of a subject.

Fig. 12 is a diagram showing a classification result of the body movement information of the subject.

Fig. 13 is a diagram showing a display example of a classification result and an alert displayed on an operation unit.

Fig. 14 is a flowchart for describing processing of body movement correction.

Figs. 15A and 15B are diagrams for describing a body movement correction function.

Fig. 16 is a diagram showing a flow of processing of another body movement information processing device.

Fig. 17 is a diagram illustrating a mask.

Fig. 18 is a diagram showing a concept of a three-dimensional mask.

Figs. 19A and 19B are diagrams for describing another indicator.

## DESCRIPTION OF THE PREFERRED EMBODIMENTS

Hereinafter, a preferred embodiment of the present invention will be described in detail with reference to the accompanying drawings. In the following description and the accompanying drawings, the same constituent elements are denoted by the same reference numerals, and the duplicated description thereof is omitted. In addition, in the following embodiment, in a case in which a plurality of constituent elements are described and listed, it can be interpreted that at least one of the plurality of constituent elements is included. Hereinafter, embodiments of a nuclear magnetic resonance imaging apparatus and a body movement information processing method according to the present invention will be described.

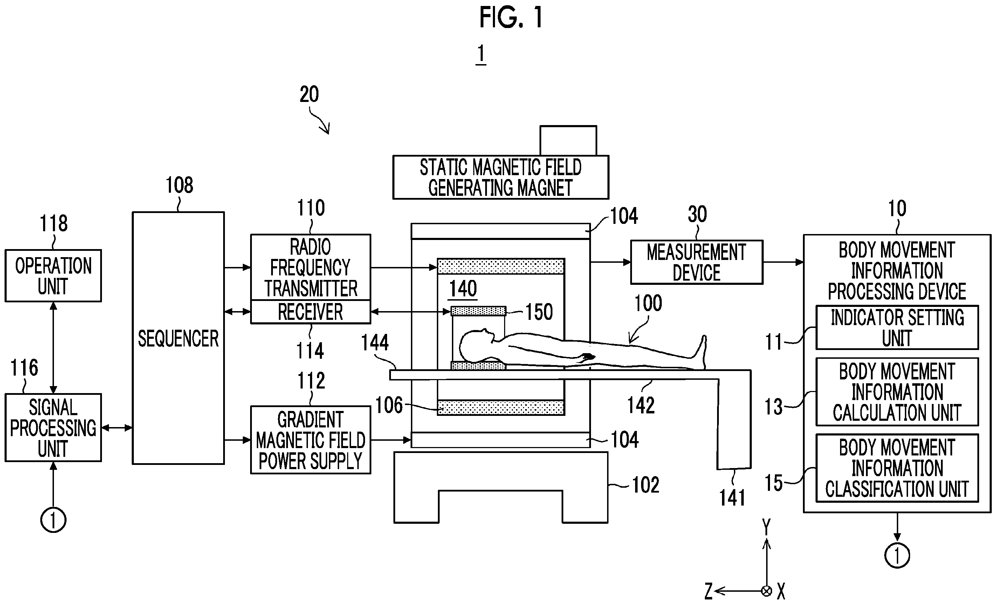

As shown in Fig. 1, a medical image capturing apparatus 1 comprises an MRI device 20 that acquires a diagnosis image of a subject, a measurement device 30 that measures movement information of a subject 100, and a body movement information processing device 10 that classifies body movement information of the subject using the movement information from the measurement device 30. The body movement information processing device 10 is an example of a body movement information processing unit according to the embodiment of the present invention. The medical image capturing apparatus 1 is an example of a nuclear magnetic resonance imaging apparatus according to the embodiment of the present invention.

A three-dimensional coordinate system illustrated in Fig. 1 shows an example of definition of directions in the MRI device 20. An X axis, a Y axis, and a Z axis of the three-dimensional coordinate system are merely examples and the present invention is not limited thereto. For ease of understanding in the following description, the X axis, the Y axis, and the Z axis in the MRI device 20 are defined to be in the same direction in all the drawings. In addition, as an example of the three-dimensional coordinate system, the Z axis direction is a static magnetic field direction and is a front-rear direction of a major axis of an imaging space 140. The Y axis direction is an up-down direction of the subject. The X axis direction is a left-right direction of the subject. The MRI device 20 is an example of an imaging unit according to the embodiment of the present invention.

The MRI device 20 is installed in an examination room of an image diagnostic facility. In the examination room, the subject 100 is placed on a top plate 144 of a table 142 of a bed device 141.

The MRI device 20 comprises a static magnetic field generating magnet 102, a gradient magnetic field coil 104, and a transmission coil 106. The subject 100 is transported toward the static magnetic field generating magnet 102 of the MRI device 20 as the top plate 144 is moved.

The static magnetic field generating magnet 102 generates a uniform static magnetic field in the imaging space 140 in which a subject 100 is disposed. The gradient magnetic field coil 104 generates a gradient magnetic field in the imaging space 140. The transmission coil 106 generates, in the imaging space 140, a radio frequency magnetic field for generating a nuclear magnetic resonance (NMR) signal (hereinafter, referred to as an NMR signal) in a nucleus of an atom constituting a tissue of the subject 100.

The MRI device 20 comprises a radio frequency transmitter 110, a receiver 114, a gradient magnetic field power supply 112, a sequencer 108, a signal processing unit 116, and an operation unit 118.

The sequencer 108 sends commands to the radio frequency transmitter 110 and the gradient magnetic field power supply 112 in accordance with an imaging sequence (pulse sequence), and appropriately amplified signals are sent to the transmission coil 106 or the gradient magnetic field coil 104.

The signal sent to the transmission coil 106 is applied to the subject 100 as a pulsed radio frequency magnetic field (RF pulse) via the transmission coil 106. The NMR signal generated from the subject 100 is detected by a coil element of a receive coil 150, and is demodulated by the receiver 114.

The gradient magnetic field coil 104 is composed of gradient magnetic field coils in three directions of X, Y, and Z, and generates gradient magnetic fields according to the signals from the gradient magnetic field power supply 112.

A nuclear magnetic resonance frequency (demodulation reference frequency f0) to be used as a reference for the demodulation in the receiver 114 is set by the sequencer 108. The sequencer 108 performs control such that each unit operates at pre-programmed timing and intensity. Among programs, a program that particularly describes the timing and intensity of RF pulses, gradient magnetic fields, and signal reception is referred to as a pulse sequence.

Various pulse sequences depending on the purpose are known, but the detailed description thereof will be omitted here.

The signal processing unit 116 controls an operation of the MRI device 20 via the sequencer 108, and receives the signal demodulated by the receiver 114 and performs various types of signal processing such as image reconstruction. The receiver 114 performs quadrature phase demodulation on the reception signal (NMR signal), which is an analog wave, using the set demodulation reference frequency f0, performs analog-to-digital (AD) conversion, and then transmits the signal to the signal processing unit 116. This data is also referred to as a reception signal or measurement data.

The signal processing unit 116 receives various instruction inputs from the operation unit 118 and integrally controls respective units of the MRI device 20. In addition, the signal processing unit 116 performs processing of performing inverse Fourier transform on the reception signal in a spatial frequency region (k space) received via the sequencer 108 to convert the reception signal into an image in a real space, and generates an MRI image.

The signal processing unit 116 is implemented by a general-purpose computer such as a personal computer or a microcomputer. The signal processing unit 116 comprises a processor (for example, a central processing unit (CPU)), a read only memory (ROM), a random access memory (RAM), an input/output interface, and the like. The processor included in the signal processing unit 116 is an example of a processor according to the embodiment of the present invention.

In the signal processing unit 116, various programs such as a control program stored in the ROM are loaded onto the RAM, and the programs loaded onto the RAM are executed by the CPU. As a result, the functions of the respective units of the MRI device 20 are implemented, and various types of arithmetic processing and control processing are executed via the input/output interface.

The operation unit 118 includes a mouse, a keyboard, a display device, a gantry monitor, and the like. The operation unit 118 functions as a part of a graphical user interface (GUI) that receives an input from the user. The MRI image or the like generated by the signal processing unit 116 is displayed on a display device of the operation unit 118.

The MRI device 20 is an example of an imaging apparatus according to the embodiment of the present invention. Although the MRI device 20 is exemplified as the imaging apparatus, an imaging apparatus such as a computed tomography (CT) apparatus, an X-ray diagnostic apparatus, and a positron emission tomography (PET) may be used. The type of the imaging apparatus is not particularly limited as long as the imaging apparatus comprises an imaging space and can generate an image of the subject.

The measurement device 30 is a device that acquires biological information of the subject 100 under an examination using the MRI device 20 in a noncontact manner. The measurement device 30 is configured of, for example, a camera. In a case in which the measurement device 30 is a camera, moving image data (time-series image data) in which the camera images a predetermined range of the subject 100 is an example of a signal of the movement information measured by the measurement device 30. The moving image data is an example of an image captured by a camera according to the embodiment of the present invention.

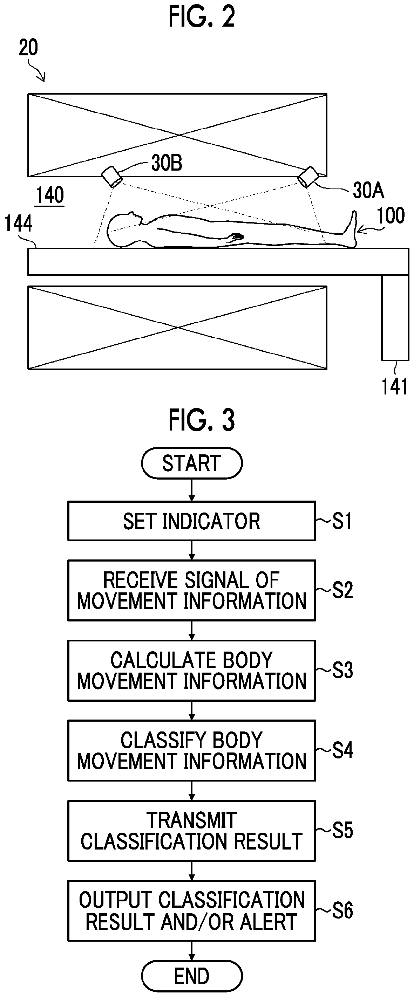

Fig. 2 shows an example in which two cameras 30A and 30B are installed as the measurement device 30 in the imaging space 140 of the MRI device 20. The top plate 144 on which the subject 100 is placed is inserted into the imaging space 140 of the MRI device 20. The camera 30A is installed upward near an insertion side end part of the imaging space 140. The camera 30B is installed above a side of the imaging space 140 opposite to the insertion side. The cameras 30A and 30B are installed at positions where the cameras 30A and 30B can capture an image of the subject 100 from diagonally above. As shown in Fig. 2, the cameras 30A and 30B are attached to both sides of the major axis of the imaging space 140, whereby it is possible to select and use moving image data suitable for detecting the movement information of the subject 100. For example, in the example shown in Fig. 2, the camera 30A on the insertion side end part is close to the abdomen of the subject 100, and the camera 30B on the opposite side is close to the face of the subject 100. The cameras 30A and 30B can easily acquire signals of the movement information.

Hereinafter, the cameras 30A and 30B will be described using a monocular camera as an example. In a case in which the cameras 30A and 30B are monocular cameras, the body movement of the subject is calculated as a relative value. The measurement device 30 may be a stereo camera. In a case of the stereo camera, three-dimensional information can be obtained from a stereo image. Therefore, a distance to the camera can be obtained, so that a body movement waveform can be obtained as an absolute value.

Returning to Fig. 1, the body movement information processing device 10 receives a signal of the movement information of the subject 100 from the measurement device 30, calculates the body movement information from the received signal, and classifies the calculated body movement information. The body movement information processing device 10 can present a classification result of the body movement information to the user and can output the classification result to the signal processing unit 116 of the MRI device 20. By knowing the classification result of the body movement information, the user can determine whether or not imaging can be continued and whether or not body movement correction using the body movement information can be performed.

The body movement information processing device 10 may classify the body movement information using at least two or more indicators. The body movement information processing device 10 may set a threshold value for the indicator in advance and classify the body movement information. The body movement information processing device 10 may decide a spatial domain to be calculated in a case of calculating the body movement information from the received signal.

In order to perform these functions, the body movement information processing device 10 of the embodiment comprises an indicator setting unit 11, a body movement information calculation unit 13, a body movement information classification unit 15, and the like. The body movement information processing device 10 can be configured of, for example, a computer comprising a memory and a processor (central processing unit (CPU)) and/or a graphic processing unit (GPU). Functions of the respective units of the body movement information processing device 10 can be implemented by the CPU or the like reading a program for implementing the functions. In addition, a part of the functions may be implemented by a programmable IC such as an application specific integrated circuit (ASIC) or a field programmable gate array (FPGA). These processors provided in the body movement information processing device 10 are an example of a processor according to the embodiment of the present invention.

Fig. 1 shows a case in which the body movement information processing device 10 is a device different from the MRI device 20, but the functions of the body movement information processing device 10 can also be installed in the signal processing unit 116 of the MRI device 20, and such a configuration is also included in the medical image capturing apparatus according to the embodiment of the present invention. Therefore, the signal processing unit 116 can have the functions of the processor of the body movement information processing device 10.

Hereinafter, an embodiment of processing of the body movement information processing device 10 will be described using a case in which the imaging apparatus is the MRI device 20 as an example. Fig. 3 is a diagram showing a flow of processing of the body movement information processing device.

First, an indicator for classifying the body movement information is decided (step S1). Specifically, the indicator setting unit 11 sets an indicator to be applied to the classification of the body movement information. Further, the indicator setting unit 11 can set a threshold value for each set indicator.

Here, the movement information of the subject 100 is information obtained from an actual movement of the subject 100. The signal is an electrical signal output from the measurement device 30 that has measured the movement information of the subject 100, and includes moving image data and the like. The body movement waveform (may be simply referred to as body movement) is information calculated by the processor provided in the body movement information processing device 10 based on the signal output from the measurement device 30, and corresponds to the movement information. The body movement information is information for classifying the body movement, and is information including at least two evaluation values corresponding to the indicator. The body movement information can be calculated based on the body movement waveform.

Next, processing in the indicator setting unit 11 will be described. The movement information of the subject 100 is calculated as the body movement information as will be described below. In a case in which the body movement information is classified based on the indicator, it is desirable that the indicator is perceived from the movement information of the subject 100. The perceived movement information of the subject 100 can be roughly classified into a magnitude of the movement, a duration of the movement, and a spatial domain in which the movement occurs, and it is preferable to set the magnitude of the body movement, the duration of the body movement, and the spatial domain in which the body movement occurs as indicators for classifying the body movement information.

By applying at least two or more types of these indicators to classify the body movement information, it is possible to determine not only the presence or absence of the movement of the subject 100 but also the type of the movement of the subject 100. For example, while imaging the subject 100, the user can know whether the movement of the subject 100 is a large movement related to safety or a small movement related to image quality of a captured image.

Next, a case in which a threshold value for an indicator is decided will be described using, as examples of the two types of indicators, the magnitude of the body movement of the subject and the duration of the body movement. The decided threshold value for each indicator is used for the classification of the body movement information described below.

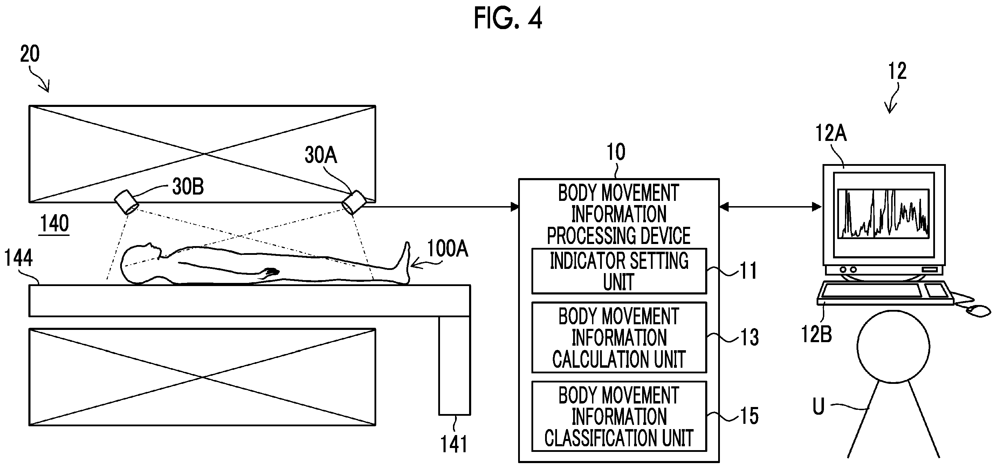

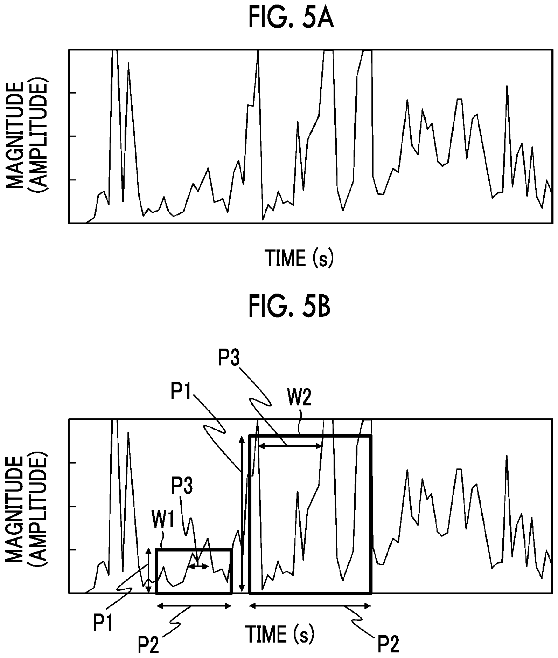

Fig. 4 is a diagram showing an example of the medical image capturing apparatus 1 for deciding the threshold value for the indicator. Figs. 5A and 5B are diagrams for describing calculation of the body movement information and setting of the threshold value.

The MRI device 20, the cameras 30A and 30B, and the body movement information processing device 10 shown in Fig. 4 are basically the same as those in Figs. 1 and 2. The body movement information processing device 10 is connected to an operation unit 12 including a display device 12A and an input device 12B. The operation unit 118 of the MRI device 20 can also serve as the operation unit 12. In the decision of the threshold value, the MRI device 20 or the like for examining the subject 100 is used. Through the application of this threshold value, it is possible to ensure the accuracy of the classification of the body movement information described below.

A subject 100A is a subject model for the body movement information processing device 10 to collect body movement information of a sample. The subject 100A reproduces a movement that may occur in the subject 100 in accordance with an instruction of a user U. The subject 100A reproduces, for example, a movement having a large change and lasting for a short time, such as coughing and sneezing, and a movement having a small change and lasting for a certain time or longer, such as wriggling of the body. In addition, the subject 100A can reproduce a movement having a large change and lasting for a certain time or longer, such as a movement related to safety, and a movement having a small change and lasting for a short time, which does not affect the imaging.

First, in a case in which signals are received from the cameras 30A and 30B, the body movement information calculation unit 13 calculates a movement vector using a known computer vision technology such as optical flow, and calculates the body movement information. The calculation of the body movement information via the body movement information calculation unit 13 will be described below. Here, a method of setting the indicator and the threshold value based on the calculated body movement information will be described.

Fig. 5A is a diagram (also referred to as a body movement) showing a body movement waveform obtained from the result of the movement vector, and is a graph in which the calculated results are plotted with a horizontal axis representing a time (s) and a vertical axis representing a magnitude (amplitude). The body movement waveform in Fig. 5A serves as a base for deciding the threshold value for the indicator. The body movement information calculation unit 13 calculates the body movement waveform and the body movement information based on an indicator suitable for the classification of the body movement information classification unit 15.

In order to calculate the body movement information from the body movement waveform of Fig. 5A, for example, waveform data including a body movement with a large amplitude and a body movement with a small amplitude in 10 seconds (101 points) is used. Next, as shown in a body movement waveform of Fig. 5B, frames (for example, a frame W1 and a frame W2) decided by a magnitude (10,000 points of 0.01 to 100) of the amplitude and a duration (80 points of 0.1 to 8 seconds) are set for the waveform, and the body movement information is extracted. For example, the graph of the waveform shown in Figs. 5A and 5B is displayed on the display device 12A of the operation unit 12 as shown in Fig. 4.

An amplitude magnitude P1 and a duration P2 are set differently between the frame W1 and the frame W2. For example, in the body movement waveform, it is determined that the body movement has occurred in a case in which a set amplitude is exceeded, and it is determined that the body movement is continuing in a case in which the set amplitude is exceeded within the set duration. In the body movement waveform, in a case in which the set amplitude is not exceeded within the set duration, it is determined that the body movement has ended. In a case in which a condition of the frame W1 is satisfied, the body movement is recognized as the body movement of one waveform, and the body movement information is extracted. Similarly, in a case in which a condition of the frame W2 is satisfied, the body movement is recognized as the body movement of another one waveform, and the body movement information is extracted. The body movement information has information on the amplitude and the duration corresponding to the indicator.

In a case of determining the duration, the amplitude of the body movement waveform may fluctuate, and the set amplitude may be exceeded or not exceeded repeatedly, so that it may be difficult to determine the duration. In this case, a time interval P3 for determining the duration is set. For example, considering a case in which the waveform has two peaks, a time from a point at which the body movement waveform exceeds the set amplitude P1 once and then falls below the set amplitude P1 to a point at which the body movement waveform exceeds the set amplitude P1 again is calculated. In a case in which the time is shorter than the time interval P3 for determining the duration, the two-peaked waveform is determined as one continuous body movement, and in a case in which the time is longer than the time interval P3, the two-peaked waveform is determined as separate body movement waveforms, not as a continuous body movement.

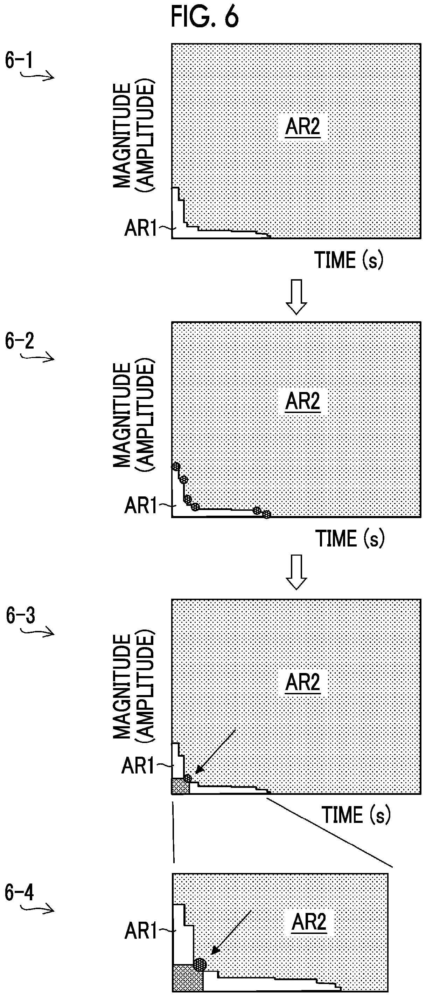

Fig. 6 is a diagram for describing a method of extracting feature points of the body movement information from the body movement information (body movement of one waveform) extracted in Figs. 5A and 5B. 6-1 of Fig. 6 is a graph in which the body movement information extracted in Figs. 5A and 5B is mapped with a vertical axis representing a magnitude (amplitude) and a horizontal axis representing a time. As shown in 6-1 of Fig. 6, in a case of performing the mapping, the body movement information can be represented by a region AR1. As a result, the graph is divided into the region AR1 and a region AR2 other than the region AR1. That is, the region AR1 corresponds to a case in which the set amplitude or duration value is exceeded, and the region AR2 corresponds to a case in which the set amplitude or duration value is not exceeded. A boundary line between the region AR1 and the region AR2 is information indicating a feature of an amplitude or a duration of a certain body movement waveform.

6-2 of Fig. 6 is a graph in which boundary points are plotted on the boundary line between the region AR1 and the region AR2. Each boundary point can be represented by a value of a product of a magnitude (amplitude) and time.

6-3 of Fig. 6 is a graph showing only a boundary point having the largest product of the magnitude (amplitude) and the time among the boundary points. 6-4 of Fig. 6 is an enlarged view of 6-3 of Fig. 6. By extracting a boundary point at which the product is maximized as a feature point with respect to the body movement information, the body movement information can be defined as one feature point having the magnitude (amplitude) and time. As a result, it is possible to exclude a case in which the magnitude (amplitude) or time is extremely small (a case in which a large amount of noise may be included). Here, the boundary point at which the product is maximized is defined as the feature point of the body movement information, but the feature point for the body movement information is not limited to this and can be selected appropriately by the user.

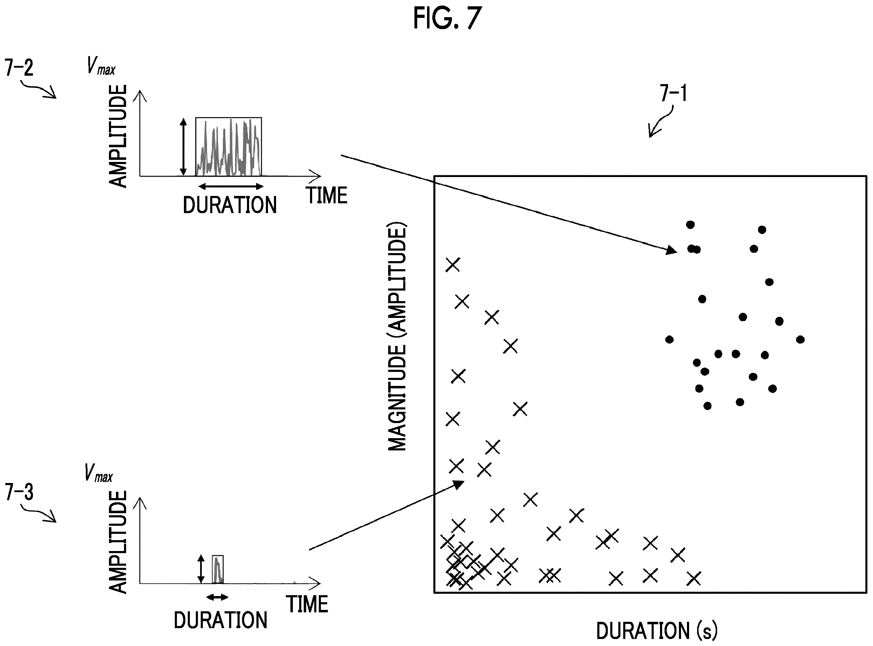

Fig. 7 is a graph (also referred to as a classification graph) in which the feature points of each of the plurality of pieces of body movement information extracted based on Figs. 5A and 6B and Fig. 6 are plotted with a vertical axis representing a magnitude (amplitude) and a horizontal axis representing a duration (s). The body movement information extracted by the body movement information calculation unit 13 is input to the indicator setting unit 11. The indicator setting unit 11 creates a classification graph shown in 7-1 of Fig. 7, for example, based on each feature point of the plurality of pieces of body movement information.

Here, **"•"** mark indicates a feature point (body movement information) corresponding to a movement in which the amplitude of the subject 100A is large and the duration is long. In addition, "×" mark indicates a feature point (body movement information) corresponding to a movement in which the amplitude of the subject 100A is large and the duration is short, a movement in which the amplitude is small and the duration is short, and a movement in which the amplitude is small and the duration is long. The body movement information of the subject 100A having a large movement (**"•"** mark) includes, for example, a movement of the subject in a range that affects the ensuring of safety in which it is difficult to continue the examination. For example, the movement is the body movement shown in the body movement waveform of 7-2 of Fig. 7, and has a large magnitude (amplitude) and a long duration.

The body movement information of the subject 100A having a small movement ("×" mark) includes a movement of the subject in a range in which the examination can be continued and the image quality of the captured image is affected but can be corrected, or a movement of the subject in a range in which the examination can be continued and the image quality of the captured image is not affected. For example, the movement is a body movement shown in the body movement waveform of 7-3 in Fig. 7, and has a small magnitude (amplitude) and a short duration.

In 7-1 of Fig. 7, only the feature points extracted in 6-3 of Fig. 6 are plotted, but all the boundary points shown in 6-2 of Fig. 6 can also be plotted.

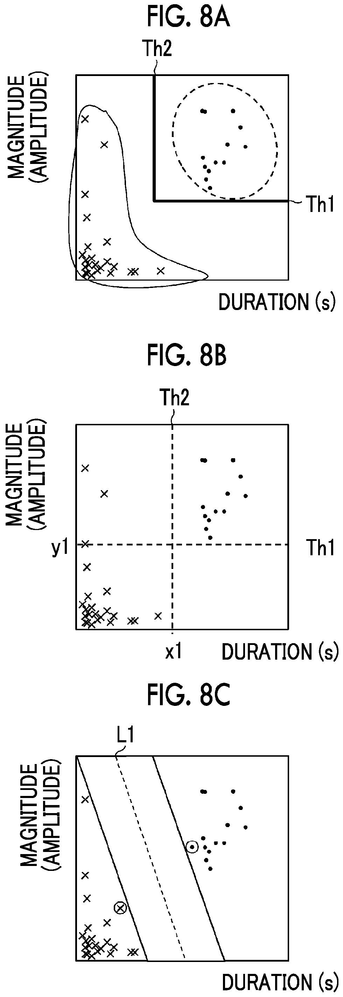

Next, a variation in which the threshold value for the indicator is set will be described with reference to Figs. 8A to 8C. The threshold value for the indicator is set by the indicator setting unit 11.

A classification graph of Fig. 8A is a diagram showing a first variation of the method of setting the threshold value, a classification graph of Fig. 8B is a diagram showing a second variation of the method of setting the threshold value, and a classification graph of Fig. 8C is a diagram showing a third variation of the method of setting the threshold value.

The method of setting the threshold value of the first variation is a method of determining the threshold value based on a rule. As shown in Fig. 8A, the body movement information can be divided into a first group of body movement information of "•" marks surrounded by a broken line and a second group of body movement information of "×" marks surrounded by a solid line. It can be understood that the first group and the second group are classified by setting a threshold value Th1 and a threshold value Th2 for the magnitude (amplitude) and the time (duration), respectively. That is, the threshold value Th1 is decided for the indicator of the magnitude (amplitude), and the threshold value Th2 is decided for the indicator of the time (duration). Since the first variation is determined by the user himself/herself, the content of the classification processing is clarified.

Next, a method of setting the threshold value using a machine learning method will be described. In the machine learning method, the threshold value is set using the extracted body movement information as correct answer data.

The method of setting the threshold value of the second variation is a case in which a decision tree, which is one of machine learning methods, is applied. The decision tree is one of machine learning methods for performing classification or the like using a tree structure, and aims to find an optimal condition for dividing given data. The decision tree has a tree structure, and the tree structure has a leaf representing classification and a branch indicating a feature amount leading to the leaf. In a case in which correct answer data is given, the decision tree algorithm finds an optimal condition for dividing the data. In the example of Fig. 8B, a condition of y1 as the magnitude (amplitude) and x1 as the duration is found by the decision tree, and y1 is decided as the threshold value Th1 and x1 is decided as the threshold value Th2. According to the decision tree, the classification accuracy can be increased from the method of setting the threshold value of the first variation, and the content of the classification processing is clarified. Since the decision tree is a known technology, detailed description thereof will be omitted.

The method of setting the threshold value of the third variation is a case in which a support vector machine (SVM), which is one of machine learning methods, is applied. The SVM aims to find a boundary line (hyperplane) for optimally classifying given data. In the SVM, a support vector is determined to decide a division line. In the SVM, non-linear data can be processed by margin maximization and a kernel method. In Fig. 8C, straight lines are drawn at positions close to a support vector of "•" mark surrounded by a circle and a support vector of "×" mark surrounded by a circle. The body movement information of "•" mark and the body movement information of "×" mark are separated by portions of the two straight lines. A straight line L1 indicated by a broken line is drawn between the two straight lines. Threshold values for two indicators can be decided based on the straight line L1 of the broken line, and the body movement information can be classified. A stepped line can also be drawn instead of the straight line L1. In the SVM, the classification accuracy can be increased from the methods of setting the threshold values of the first and second variations, and the content of the classification processing is clarified. Since the SVM is a known technology, detailed description thereof will be omitted.

In the methods of setting the threshold value of the second variation and the third variation, in a case in which the body movement information of the correct answer data is insufficient, augmented correct answer data of the body movement information may be generated by data augmentation. For example, the data may be augmented in the direction of the magnitude of the body movement within a possible range, or the data may be augmented in the direction of the duration of the body movement within a possible range. In this case, for example, data in which the magnitude of the body movement is 0.5 times, 0.6 times, ..., 1.5 times is generated, or data in which the duration is doubled or tripled is generated. In a case in which the body movement information is set as the correct answer data including the augmented correct answer data, the classification accuracy is increased. Since the data augmentation is a known technology, description thereof will be omitted.

The threshold value decided by the first to third variations is stored in the memory or the like provided in the body movement information processing device 10, and the setting of the threshold value is completed. The threshold value is used for classifying the body movement information.

In the embodiment, a case in which the threshold value is decided by a machine learning function implemented in the indicator setting unit 11 has been described, but the present invention is not limited thereto. For example, the machine learning function may be implemented in another system, and the threshold value may be calculated in a case in which the body movement information is input. That is, it is sufficient for the indicator setting unit 11 to be able to select the indicator and set the threshold value in response to an instruction from the user or automatically. In addition, the machine learning may be performed in advance before the product is shipped, and the threshold value calculated by the machine learning may be held in the indicator setting unit 11.

After the indicator is set (step S1), the subject 100 is imaged using the medical image capturing apparatus 1. In a case in which the indicator is set, in the subsequent examination, the indicator setting (step S1) can be omitted, and the subject 100 can be examined.

As shown in Fig. 3, after the indicator is installed (step S1), a signal of the movement information is received (step S2). Specifically, while an MRI image of the subject 100 is being captured, the body movement information calculation unit 13 receives a signal of the moving image data of the subject 100 imaged by the cameras 30A and 30B.

After receiving the signal of the moving image data (step S2), the body movement information is calculated (step S3). Specifically, the body movement information calculation unit 13 calculates the movement from a movement vector and a velocity vector to calculate the body movement.

After receiving the signal of the moving image data, the body movement information calculation unit 13 calculates the movement vector. The movement vector can be calculated using, for example, a known computer vision technology such as optical flow. The optical flow calculates a movement vector for each pixel of the image from a temporal change of the moving image data. As an optical flow algorithm, for example, a Ferneback method and a Lucas-Kanade method are known, and any of these methods may be employed.

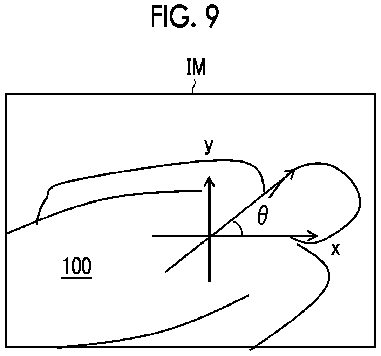

The calculated result is a representation of a change in pixel position between frames in terms of a velocity vector for each pixel. For example, as shown in Fig. 9, a velocity in the x direction (Vx) and a velocity in the y direction (Vy) are calculated in a case in which the vertical and horizontal directions of an image IM are the x direction and the y direction.

The body movement is calculated by the following expression using the velocity vectors Vx and Vy. In this case, in a case in which the subject 100 is in a posture of lying on the table, the movement of respiration of the subject 100 is mainly a movement perpendicular to a horizontal plane (table surface). On the other hand, there is a difference in the characteristics of the two movements that the movement of the subject 100 other than the movement of respiration is in an irregular (or random) direction. It is possible to perform calculation using a different calculation expression depending on the body movement type. For example, the body movement type can be determined based on information on an examination site in a case in which the user sets the examination site at the start of the examination.

For the sudden movement, for example, Vmotion can be calculated by Expression (1) or Expression (2).

$$V_{motion} = (V_x sin\theta + V_y cos\theta)^2 \qquad (1)$$

$$V_{motion} = (V_x sin\theta)^2 + (V_y cos\theta)^2 \qquad (2)$$

In a case in which it is desired to observe the body movement (respiratory movement) in the movement direction of the chest with high sensitivity, Vabd_motion can be calculated by Expression (3), or Vmotion can be calculated by Expression (4) using Expression (3).

$$V_{abd\_motion} = V_x cos\theta - V_y sin\theta \qquad (3)$$

$$V_{motion} = \left| \sum_{t-tcount}^{t} V_{abd\_motion}(t) \right| \qquad (4)$$

According to Expression (4), by integrating the number of data items corresponding to tcount in the time direction, the effect of improving the sensitivity is achieved. In the expression, $\theta$ indicates an angle between the horizontal direction and a direction of the respiratory movement (also referred to as an abdomen-back direction or an anterior (A)-posterior (P) direction). In this example, the velocity components in the horizontal direction and the vertical direction are calculated by the optical flow, and $\theta$ is set based on information on the velocity components to increase detection sensitivity of the respiratory movement.

In addition, $\theta$ in the expression is an angle of an XY plane with respect to the horizontal plane, and varies depending on a positional relationship with the cameras 30A and 30B, an attachment angle of the cameras, and the like. In addition, $\theta$ may be determined after analyzing the direction of the respiratory movement for each subject. The respiratory movement and the movement or the body movement can be appropriately set to be detected with high sensitivity, and different values may be used for each position (pixel). The expression representing the body movement need only represent the body movement to be detected, and is not limited to (1) to (4).

The body movement information calculation unit 13 can resize the image of the moving image data and downsize an original image size as necessary. For example, an original size of a camera image is reduced to about 1/2 to 1/10. By reducing the size of the image of the moving image data in this way, it is possible to reduce a computational load for the subsequent processing such as the calculation of the movement vector, which allows for computational acceleration (real-time presentation). Further, in a subsequent processing, in order to divide one image into a plurality of regions having the same pixel size, image end parts of the resized image, which are remainders, may be cut such that both the height and the width are divisible by the number of divisions.

The resized image is divided into small regions, and an average value of the velocity vector and the movement vector obtained for each pixel in the small region is calculated for each divided small region. As an example, in a case in which a pixel size of a resized camera image of which the remainders are removed is 720 in width × 540 in height, a size of one small region obtained by dividing the camera image into 60 regions is 72 × 90 pixels, and an average value of the velocity vector and the movement vector is calculated for each small region having such a size.

The present invention is not limited to the optical flow, and the body movement information can also be calculated from a temporal change in a correlation coefficient of an image captured by a camera. For example, first, a target image data is acquired. Next, a correlation coefficient is calculated for consecutive image data (frames). Next, the correlation coefficient can be calculated in units of pixels. Relevance between the corresponding pixels is evaluated. A temporal change in the calculated correlation coefficient is analyzed, and the change is detected. The body movement waveform may be calculated based on the change in the correlation coefficient.

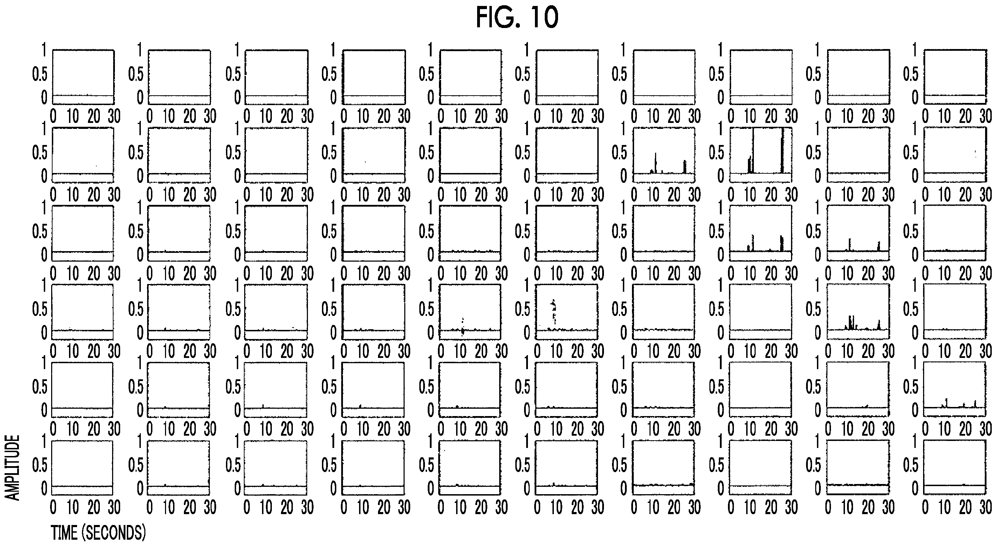

Fig. 10 is a diagram showing an example of a body movement and body movement information calculated by the body movement information calculation unit 13 based on the movement vector. Each body movement waveform in Fig. 10 is a graph calculated for each small region, with a vertical axis representing a magnitude (amplitude) and a horizontal axis representing a time. Each body movement waveform is calculated by the body movement information calculation unit 13 based on the moving image data obtained by imaging the entire subject 100 with the cameras 30A and 30B. In this example, the body movements of 60 small regions are calculated.

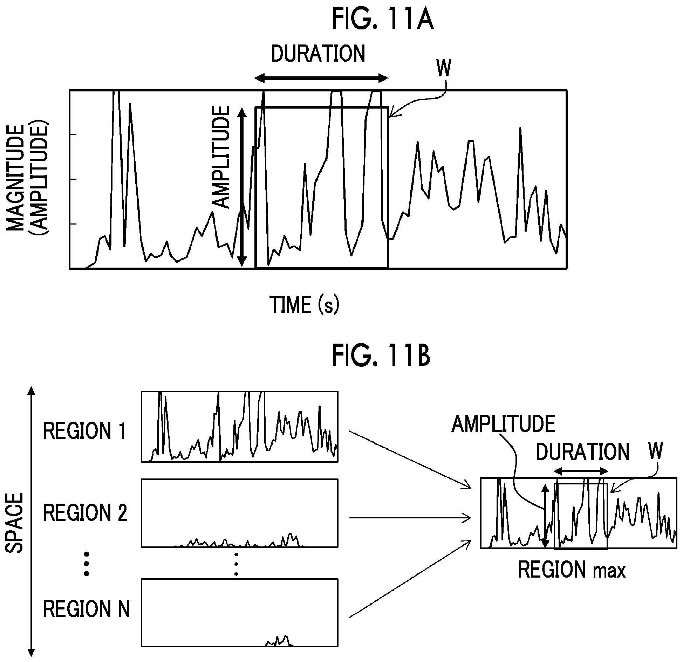

Next, a method of calculating the body movement information according to the indicator will be described with reference to Figs. 11A and 11B. Fig. 11A shows an example in which the body movement information is calculated using two indicators (amplitude and duration), and Fig. 11B shows an example in which the body movement information is calculated using three indicators (amplitude, time, and space).

Fig. 11A is a body movement waveform in which the body movement information calculation unit 13 calculates a body movement of a designated region of the subject 100. That is, a partial region of the subject 100 is a monitoring target. The body movement information calculation unit 13 calculates the body movement information using the amplitude and the duration as the indicators. The calculated body movement information is classified by the body movement information classification unit 15. The body movement information calculation unit 13 calculates the body movement information by using the body movement waveform surrounded by a frame W as one waveform.

In Fig. 11B, the body movement information calculation unit 13 simultaneously calculates body movement waveforms of a plurality of regions (spaces) 1, 2, ..., and N of the subject 100. That is, in Fig. 11B, the body movement waveform is calculated using the space as one of the indicators. In a case in which the body movement waveforms of the plurality of regions are simultaneously calculated, it is important to decide from which region's body movement waveform the body movement information is calculated. As one decision method, in Fig. 11B, the maximum value (maximum amplitude) of all regions is selected at each time point, and the body movement waveform is calculated. A region max is a body movement waveform in which the maximum amplitude is plotted for each time. That is, the region max is a body movement waveform in which the characteristic movement of the subject 100 is selected. Therefore, the region max is a composite body movement waveform including the movements of the plurality of regions of the subject 100. As another decision method, one region in which the body movement is large may be selected based on a body movement waveform for a certain time.

Next, similarly to Fig. 11A, the body movement information calculation unit 13 calculates the body movement information from the region max using the amplitude and the duration as the indicators. The body movement information calculation unit 13 calculates the body movement information by using the body movement waveform surrounded by a frame W as one waveform. The calculated body movement information is classified by the body movement information classification unit 15.

As another decision method, any one region may be selected as a representative from a plurality of regions (spaces) 1, 2, ..., and N. The user can appropriately set a region to be monitored depending on a site to be imaged.

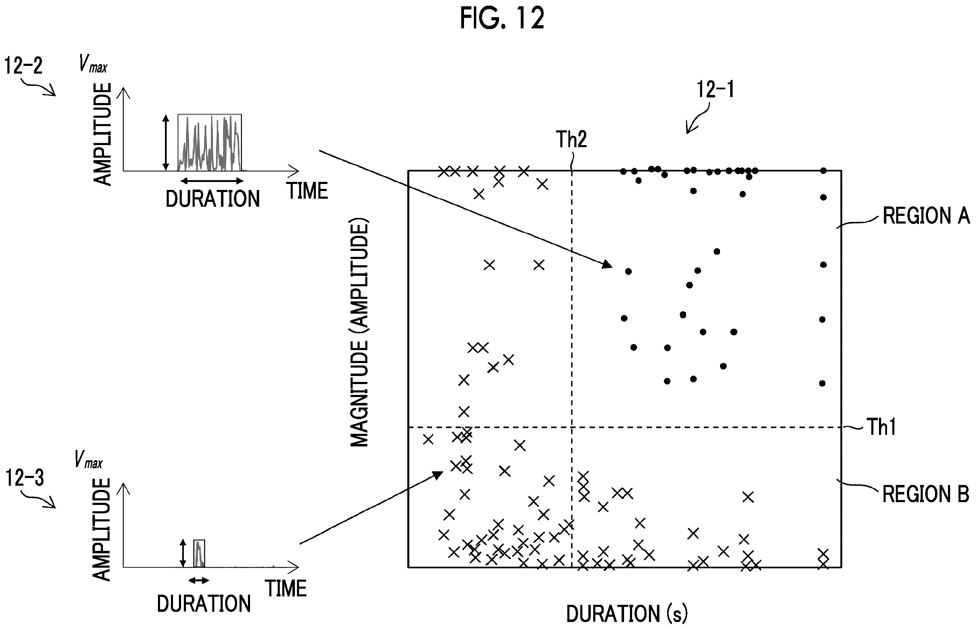

After calculating the body movement information (step S3), the body movement information is classified (step S4). Specifically, the body movement information classification unit 15 classifies the body movement information (including information regarding the amplitude and the duration) based on the indicator. Fig. 12 shows a result (classification result) of the body movement information classified by the body movement information classification unit 15. In 12-1 of Fig. 12, the body movement information classified regardless of an occurrence time of the body movement is plotted on a graph with a vertical axis representing a magnitude (amplitude) and a horizontal axis representing a time. Further, threshold values Th1 and Th2 set by the indicator setting unit 11 are displayed. The body movement information ("•" mark) of an upper right region A (amplitude equal to or greater than the threshold value Th1 and duration equal to or greater than the threshold value Th2) surrounded by the threshold values Th1 and Th2 corresponded to a large movement of the subject 100 as shown in 12-2 of Fig. 12. In addition, the body movement information ("×" mark) of a region B (amplitude less than the threshold value Th1 or duration less than the threshold value Th2) other than the region A corresponded to a small movement of the subject 100 as shown in 12-3 of Fig. 12.

According to the classification result, it can be understood that the body movement information of the subject 100 can be classified by the magnitude (amplitude) of the body movement of the threshold value Th1 and the duration of the body movement of the threshold value Th2.

Next, after classifying the body movement information (step S4), and the classification result is transmitted (step S5). Specifically, the body movement information processing device 10 transmits the calculated body movement waveform and the classification result of the body movement information to the signal processing unit 116 of the MRI device 20. The body movement waveform includes a time point at which the body movement occurs and a magnitude of an amplitude.

Next, after transmitting the classification result (step S5), the classification result and/or an alert is output (step S6). Specifically, the signal processing unit 116 outputs the calculated body movement and the classification result of the body movement information to a display device (not shown) of the operation unit 118. The signal processing unit 116 can output these pieces of information to a printing device or a storage device in addition to the display device.

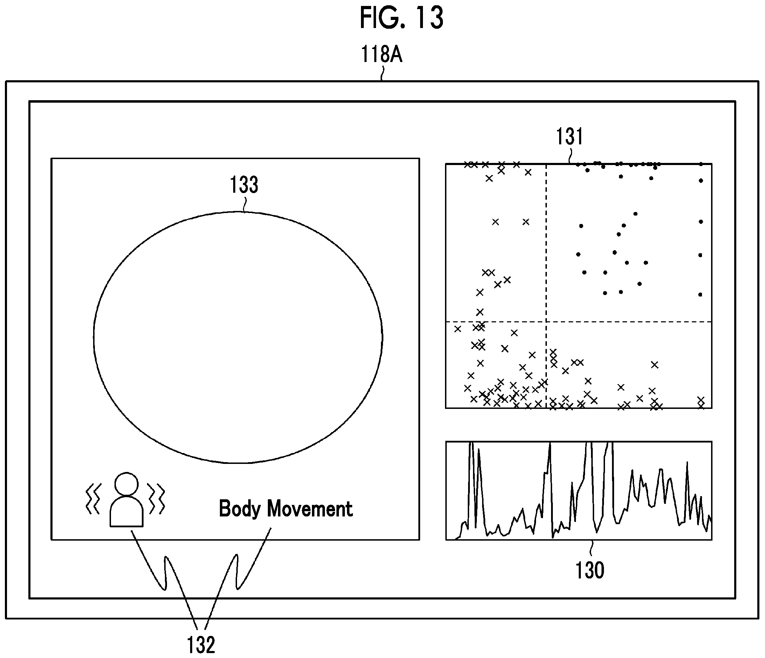

Fig. 13 is a diagram showing a display example of a body movement waveform 130, a classification result 131, and an alert 132 displayed on a display device 118A of the operation unit 118. As shown in Fig. 13, an image 133 captured by the MRI device 20 is displayed on the display device 118A. Further, the body movement waveform 130 and the classification result 131 are displayed. The body movement waveform 130 and the classification result 131 are associated with the occurrence time. Therefore, the user can know at what time the body movement information on the classification result 131 is caused by the body movement.

The method of outputting the alert 132 is not particularly limited, but in this example, the alert 132 is displayed below the image 133, for example, with an icon and text. The alert 132 may be any one of an icon or text. In addition, the alert 132 can change the display of the icon and the text (size, color, content of the text, and the like) depending on the classification. At the same time, a warning sound may be emitted, or an alert may be issued by voice. In addition, these may be combined as appropriate.

Depending on the classification, the alert 132 can be output to inform a user who is concentrating on other tasks that an abnormal movement has occurred. In addition, it is possible to continue the imaging from the viewpoint of the safety of the subject as in a case in which the subject sneezes or temporarily coughs depending on the classification, and it is possible to avoid unnecessary interruption of the imaging. On the other hand, in a case in which the imaging has to be interrupted from the viewpoint of the safety of the subject, the user can avoid danger such as falling down of the subject.

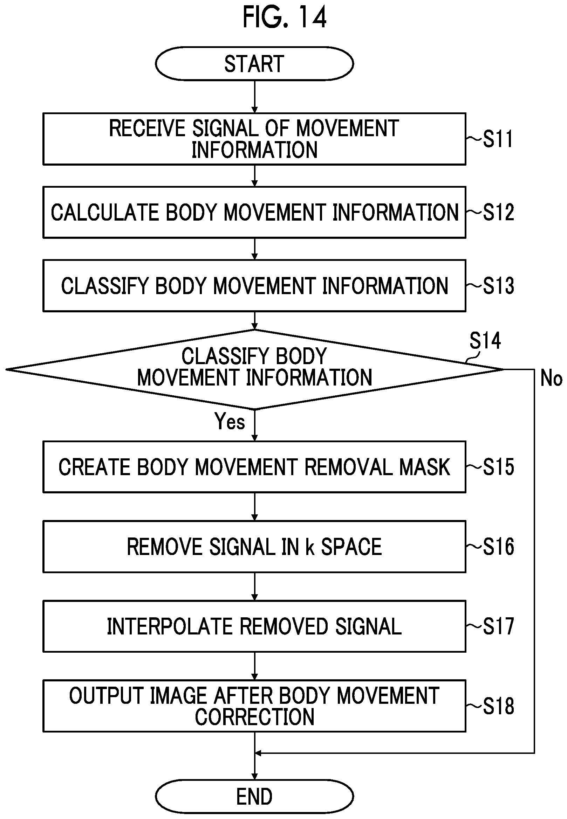

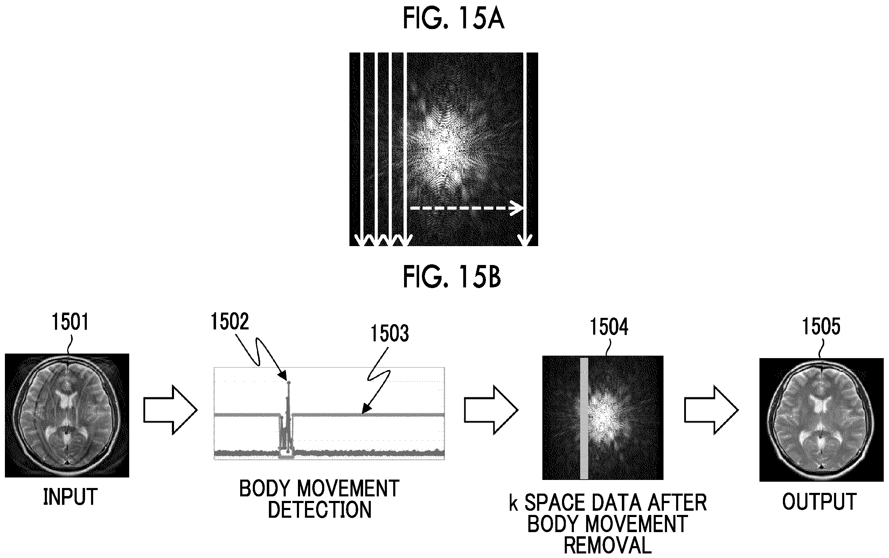

Next, for the use of the body movement waveform and the classification result of the body movement information, body movement correction other than the alert will be described with reference to Fig. 14 and Figs. 15A and 15B. Fig. 14 is a flowchart for describing processing of body movement correction. Figs. 15A and 15B are diagrams for describing a body movement correction function. Description of a part common to the above-described processing flow may be omitted. Here, the body movement correction means reconstructing an image in which an artifact of the generated MRI image is reduced.

As shown in Fig. 14, a signal of the movement information is received (step S11), the body movement information is calculated (step S12), and then the body movement information is classified (step S13). Specifically, the same processes as in step S1, step S2, and step S3 are executed.

The classification result is transferred to the signal processing unit 116, and it is determined whether the body movement can be corrected (step S14). The signal processing unit 116 can determine whether or not the body movement correction is possible based on the classification result. In the determination, a preset threshold value is used for the indicator. The signal processing unit 116 comprehensively determines an amplitude and a duration, which are threshold values, to determine whether or not the body movement correction is possible.

In a case in which the amplitude is small and the image quality is not affected even though a correction function is not executed, the correction function is not executed. In addition, in a case in which the amplitude is large and the image quality is not improved even though the correction function is executed, the correction function is not executed. The present invention is not limited to these cases, and the signal processing unit 116 can decide whether or not the body movement correction is possible.

In a case in which it is determined that the body movement correction cannot be performed (No in step S14), the processing of the body movement correction is not executed and the process ends. After the end, the MRI image without the body movement correction is reconstructed, or no MRI image is reconstructed.

On the other hand, in a case in which it is determined that the body movement correction can be performed (Yes in step S14), the processing of the body movement correction is executed.

As shown in Fig. 15A, in the MRI device 20, an MRI image of a subject is generated by filling a measurement data space (k space) with measurement lines (indicated by a solid line arrow) according to a certain measurement order (indicated by a broken line arrow).

As shown in Fig. 15B, an MRI image 1501 for which it is determined that the body movement correction is possible is input to the signal processing unit 116.

Next, a body movement removal mask is created (step S15). Specifically, as shown in Fig. 15B, a body movement removal mask 1503 is created by the signal processing unit 116 based on a body movement waveform 1502 calculated by the camera. The body movement waveform 1502 indicates a body movement waveform while the MRI image is being captured. The body movement removal mask 1503 extracts a body movement that causes the artifact. The body movement removal mask 1503 is created in consideration of the amplitude, the duration, and the like of the body movement.

Next, a signal is removed in the k space (step S16). Specifically, as shown in Fig. 15B, k space data 1504 is generated in which the measurement lines collected at a time point at which the body movement waveform is generated are removed after being converted into the k space information.

Next, the removed signal is interpolated (step S17). For example, the removed signal is interpolated by applying a known interpolation method such as sequential reconstruction that performs repeated calculation while maintaining the consistency of the measurement data.

Finally, the image after the body movement correction is output (step S18). As shown in Fig. 15B, an artifact-reduced MRI image 1505 is reconstructed and output. As a result, it is possible to improve the image quality of the MRI image. In a case of creating the body movement removal mask, the body movement correction with higher accuracy can be performed by referring to the classification result of the body movement information. The artifact-reduced MRI image 1505 is an example of a body movement-corrected image according to the embodiment of the present invention.

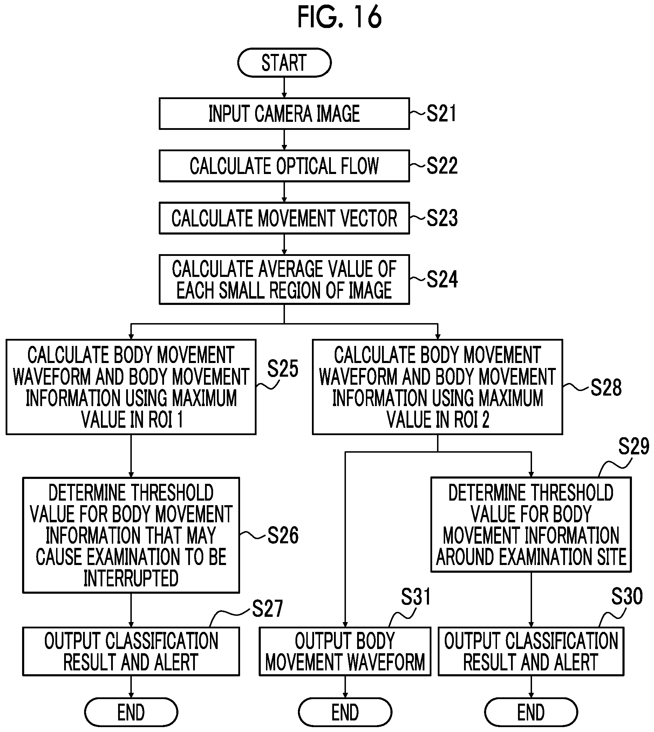

Next, a case in which body movement information is used for each different region of interest (ROI) for the movement information of the subject 100 using the medical image capturing apparatus 1 will be described with reference to Fig. 16. Description of a part common to the above-described processing flow may be omitted.

As shown in Fig. 16, the moving image captured by the cameras 30A and 30B is input to the body movement information processing device 10 (step S21). In this example, the indicator and the threshold value have already been set.

Next, the body movement information calculation unit 13 calculates the optical flow based on the signal of the moving image (step S22), and calculates the movement vector (step S23). An average value of the velocity vectors of each small region of the image is calculated from the movement vector (step S24).

Next, the body movement waveform and the body movement information are calculated using the maximum value in an ROI 1 (step S25), and the body movement waveform and the body movement information are calculated using the maximum value in an ROI 2 (step S28). Step S25 and step S28 can be executed in parallel. The ROI 1 is a region for monitoring a large body movement related to the safety, and is, for example, the entire imaging section or the entire subject. On the other hand, the ROI 2 is a region for monitoring a small body movement related to the image quality, and is an imaging region of the MRI device or an examination site (head, abdomen, joint part, or the like) of the subject.

Next, a threshold value for the body movement information (movement of the subject) that may cause the examination to be interrupted is determined (step S26). The classification result of the body movement information classification unit 15 can be used. As a result of the determination, in a case in which the threshold value is exceeded, the classification result and the alert are output (step S27). The safety of the subject can be ensured by the user checking this output.

In parallel with step S25, a threshold value for the body movement information around the examination site is determined (step S29). As in step S26, the classification result of the body movement information classification unit 15 can be used. As a result of the determination, in a case in which the threshold value is exceeded, the classification result and the alert are output (step S30). Since the ROI 2 is a region in which the subject is imaged, the body movement waveform is output regardless of the determination result (step S31). The processes of step S25 and step S29 are executed in parallel, but, in step S25, the region (ROI 1) related to the safety is monitored. Therefore, the output of the alert in step S27 is executed with priority over the alert in step S30.

Although a case in which the body movement waveform is calculated for each ROI has been described, the mask can be used to extract the body movement information in order to classify the body movement information. The body movement information processing device 10 can generate the mask in advance in response to an instruction from the user. A plurality of regions for measuring the movement information of the subject 100 can be decided. The user can cause the body movement information processing device 10 to generate the mask in advance from the operation unit 118 of the MRI device 20 via the signal processing unit 116. Even in this case, it is possible to decide a plurality of regions for measuring the movement information of the subject 100.

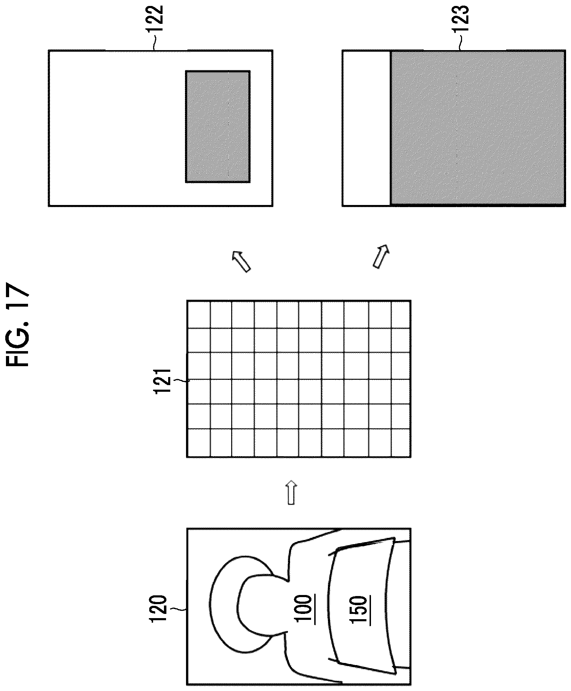

An example of a mask for extracting two regions is shown in Fig. 17. Fig. 17 shows an example in which two types of masks 122 and 123 are applied to a region map 121 of the body movement calculated for each small region of a camera image (after resizing) 120. Of these, the mask 122 extracts the imaging region and corresponds to the ROI 2. The MRI device 20 can use a mask in which a region of the subject 100 on which the receive coil 150 is mounted is set to 1 and the rest is set to 0. A position where the receive coil 150 is mounted may be determined by checking the images of the cameras 30A and 30B, or may be decided based on a center position of the imaging space and a size of the receive coil 150 since the subject 100 is disposed such that a center position of the receive coil 150 is generally near the center of the imaging space in the MRI device 20.

On the other hand, the mask 123 includes the imaging region and extracts a region wider than the imaging region, and corresponds to the ROI 1. A mask in which a region in which the subject 100 is present is set to 1 and the rest is set to 0 can be used.

By monitoring the body movement of the region extracted with the mask 122, it is possible to detect the body movement that directly affects the imaging and to feed back the information to the imaging. In addition, by monitoring the body movement of the region extracted with the mask 123, it is possible to detect any significant movement of the subject 100 during the examination even though the movement does not directly affect the imaging.

Note that the mask to be generated is not limited to the above-described example, and one or a plurality of (two or more) regions may be set in consideration of the examination site, the ease of the movement of the subject 100 (such as a child), and the like. For example, in a case of a receive coil in which a plurality of small coils are connected, a region where the receive coil is mounted may be divided into a plurality of regions, or may be divided into a region on a head side including the receive coil, a region on a leg part side including the receive coil, and the like.

Further, the mask need not be a 1/0 binary mask, and may be weighted with a predetermined weight in consideration of a positional relationship with the camera, a sensitivity distribution of the receive coil, and the like. For example, the farther the distance from the cameras 30A and 30B is, the larger the weight may be assigned because the body movement is captured smaller, and the closer the distance from the cameras 30A and 30B is, the smaller the weight may be assigned. Further, since the sensitivity of the receive coil 150 is generally high at the center and it is considered that the influence of the body movement at that portion increases, the weight may be increased at the center, and the weight may be decreased in the periphery.

In the above description, an example of a spatial mask has been described, but a temporal element and the magnitude of the body movement may further be added. The temporal element is, for example, a temporal mask indicating whether it is at the time of imaging or during the examination including before and after the imaging.

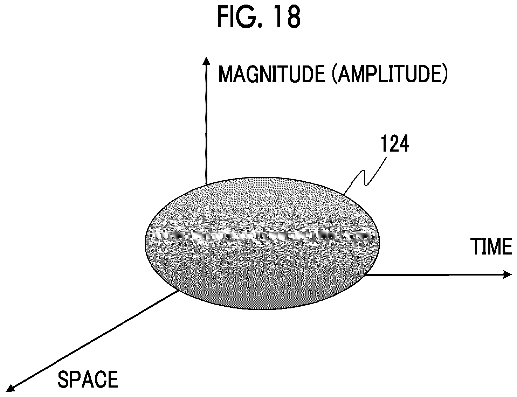

Fig. 18 shows a concept of a three-dimensional mask 124 in which the temporal element and the magnitude of the body movement are combined. By using such a three-dimensional mask, it is possible to monitor an appropriate region. Specifically, a three-dimensional mask in which a mask for selecting a sensitivity distribution region of the receive coil as a spatial mask, a mask for selecting an imaging time as a temporal mask, and a mask for selecting a first range as a mask for the magnitude of the amplitude are combined can be used. As a result, the three-dimensional mask can be used as a mask corresponding to the ROI 2. In addition, a three-dimensional mask in which a mask for selecting the entire imaging space as the spatial mask, a mask for selecting "during the examination" as the temporal mask, and a mask for selecting a second range that is equal to or greater than the first range as the mask of the magnitude of the amplitude are combined can be used. As a result, the three-dimensional mask can be used as a mask corresponding to the ROI 1.

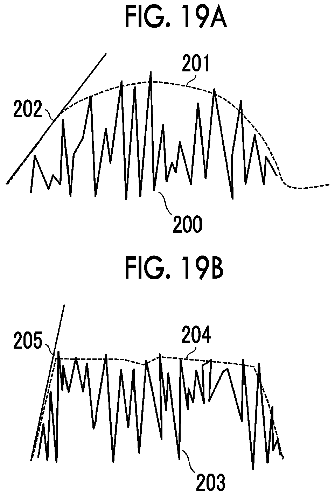

In this example, the body movement information is calculated using the magnitude (amplitude) and the duration as the indicators from the body movement waveform. A case in which an envelope curve is used as another information will be described with reference to Figs. 19A and 19B. Fig. 19A shows a body movement waveform 200, an envelope curve (envelope) 201, and a straight line 202. In addition, Fig. 19B shows a body movement waveform 203, an envelope curve 204, and a straight line 205 different from those in Fig. 19A.

The body movement waveform 200 indicates a waveform having a small rising edge. The envelope curve 201 is a line that surrounds the outside of the body movement waveform 200 and has a gentle mountain shape. The straight line 202 is in contact with the envelope curve 201.

On the other hand, the body movement waveform 203 indicates a waveform having a large rising edge. The envelope curve 204 is a line that surrounds the outside of the body movement waveform 203 and has a steep inclination. The straight line 205 is in contact with the envelope curve 204.

The inclinations of the straight lines 202 and 205 can be calculated from the envelope curves 201 and 204, and values of the inclinations can be used for the classification of the body movement information.

Further, it is needless to say that the present invention is not limited to the embodiment described above, and that various modifications are possible.

Explanation of References

1: medical image capturing apparatus
10: body movement information processing device
11: indicator setting unit
13: body movement information calculation unit
15: body movement information classification unit
20: MRI device

30: measurement device
30A, 30B: camera
100, 100A: subject

**Claims**

1. A magnetic resonance imaging apparatus comprising:

    an imaging unit (20) that measures a magnetic resonance signal generated by a subject and that acquires an image of the subject; and
    a processor that processes movement information of the subject disposed on an imaging apparatus,
    wherein the processor is configured to

       receive a signal from a measurement device (30) that measures the movement information of the subject and calculates body movement information from the signal, and classify the body movement information.

2. The magnetic resonance imaging apparatus according to claim 1,
   wherein the processor classifies the body movement information based on at least two or more types of indicators.

3. The magnetic resonance imaging apparatus according to claim 2,
   wherein the at least two or more types of indicators include a magnitude of a body movement and a duration of the body movement.

4. The magnetic resonance imaging apparatus according to claim 3,
   wherein the at least two or more types of indicators include the magnitude of the body movement, the duration of the body movement, and an envelope curve.

5. The magnetic resonance imaging apparatus according to claim 3 or 4,
   wherein the at least two or more types of indicators further include a spatial domain in which the body movement occurs.

6. The magnetic resonance imaging apparatus according to any one of claims 2 to 5,
   wherein the processor selects a partial region of the subject or a characteristic movement of the subject in a case of calculating the body movement information.

7. The magnetic resonance imaging apparatus according to any one of claims 1 to 6,

wherein the measurement device (30) is a camera, and the signal is an image captured by the camera, and the processor calculates the body movement information from a temporal change in the image,
or,
wherein the measurement device (30) is a camera, and the signal is an image captured by the camera, and the processor calculates the body movement information from a temporal change in a correlation coefficient of the image.

8. The magnetic resonance imaging apparatus according to any one of claims 1 to 6,

wherein the measurement device (30) is a stereo camera, and the signal is a stereo image captured by the stereo camera, and
the processor calculates the body movement information including three-dimensional information from the stereo image.

9. The magnetic resonance imaging apparatus according to any one of claims 2 to 5, wherein the processor sets a threshold value for each of the at least two or more types of indicators, and classifies the body movement information based on the threshold value.

10. The magnetic resonance imaging apparatus according to claim 9,

wherein the threshold value is a value decided by machine learning using previously collected body movement information as correct answer data, and
preferably, an algorithm used for the machine learning includes a support vector machine or a decision tree.

11. The magnetic resonance imaging apparatus according to claim 10, wherein the correct answer data includes augmented correct answer data generated by data augmentation.

12. The magnetic resonance imaging apparatus according to any one of claims 1 to 11, wherein the processor applies a different calculation expression depending on a movement of the subject in a case of calculating the body movement information.

13. The magnetic resonance imaging apparatus according to any one of claims 1 to 12, wherein the processor outputs a classification result and/or outputs an alert based on the classification result.

14. The magnetic resonance imaging apparatus according to any one of claims 1 to 13,

wherein the processor decides whether or not to apply a body movement correction function based on a classification result,
and/or,
wherein the processor applies a body movement correction function based on a classification result, and outputs a body movement-corrected image based on the body movement information.

15. A body movement information processing method of processing movement information of a subject disposed on an imaging apparatus (20) including a processor, the body movement information processing method comprising:
causing the processor to

receive a signal from a measurement device (30) that measures the movement information of the subject,
calculate body movement information from the signal, and
classify the body movement information.

# FIG. 1

# FIG. 2

# FIG. 3

START

SET INDICATOR ~S1

RECEIVE SIGNAL OF MOVEMENT INFORMATION ~S2

CALCULATE BODY MOVEMENT INFORMATION ~S3

CLASSIFY BODY MOVEMENT INFORMATION ~S4

TRANSMIT CLASSIFICATION RESULT ~S5

OUTPUT CLASSIFICATION RESULT AND/OR ALERT ~S6

END

FIG. 4

## FIG. 5A

## FIG. 5B

## FIG. 6

6-1

6-2

6-3

6-4

FIG. 7

## FIG. 8A

## FIG. 8B

## FIG. 8C

FIG. 9

# FIG. 10

AMPLITUDE

TIME (SECONDS)

EP 4 603 856 A1

FIG. 11A

FIG. 11B

FIG. 12

FIG. 13

EP 4 603 856 A1

## FIG. 14

```
        ┌─────────────────┐
        │      START      │
        └────────┬────────┘
                 │
    ┌────────────▼────────────┐
    │ RECEIVE SIGNAL OF       │ ~S11
    │ MOVEMENT INFORMATION    │
    └────────────┬────────────┘
                 │
    ┌────────────▼────────────┐
    │ CALCULATE BODY MOVEMENT │ ~S12
    │ INFORMATION             │
    └────────────┬────────────┘
                 │
    ┌────────────▼────────────┐
    │ CLASSIFY BODY MOVEMENT  │ ~S13
    │ INFORMATION             │
    └────────────┬────────────┘
                 │
              ◇ S14
       CLASSIFY BODY              No
       MOVEMENT INFORMATION ──────┐
              │ Yes               │
    ┌─────────▼────────────┐      │
    │ CREATE BODY MOVEMENT │ ~S15 │
    │ REMOVAL MASK         │      │
    └─────────┬────────────┘      │
              │                   │
    ┌─────────▼────────────┐      │
    │ REMOVE SIGNAL IN     │ ~S16 │
    │ k SPACE              │      │
    └─────────┬────────────┘      │
              │                   │
    ┌─────────▼────────────┐      │
    │ INTERPOLATE REMOVED  │ ~S17 │
    │ SIGNAL               │      │
    └─────────┬────────────┘      │
              │                   │
    ┌─────────▼────────────┐      │
    │ OUTPUT IMAGE AFTER   │ ~S18 │
    │ BODY MOVEMENT        │      │
    │ CORRECTION           │      │
    └─────────┬────────────┘      │
              │◄──────────────────┘
        ┌─────▼─────┐
        │    END    │
        └───────────┘
```

## FIG. 15A

## FIG. 15B

1501
INPUT

1502    1503
BODY MOVEMENT
DETECTION

1504
k SPACE DATA AFTER
BODY MOVEMENT
REMOVAL

1505
OUTPUT

# FIG. 16

```
                    ( START )
                        │
                        ▼
        ┌──────────────────────────────┐
        │     INPUT CAMERA IMAGE        │─── S21
        └──────────────────────────────┘
                        │
                        ▼
        ┌──────────────────────────────┐
        │    CALCULATE OPTICAL FLOW     │─── S22
        └──────────────────────────────┘
                        │
                        ▼
        ┌──────────────────────────────┐
        │   CALCULATE MOVEMENT VECTOR   │─── S23
        └──────────────────────────────┘
                        │
                        ▼
        ┌──────────────────────────────┐
        │   CALCULATE AVERAGE VALUE OF  │─── S24
        │   EACH SMALL REGION OF IMAGE  │
        └──────────────────────────────┘
```

```
┌──────────────────────────┐              ┌──────────────────────────┐
│  CALCULATE BODY MOVEMENT  │              │  CALCULATE BODY MOVEMENT  │
│ WAVEFORM AND BODY MOVEMENT│── S25        │ WAVEFORM AND BODY MOVEMENT│── S28
│  INFORMATION USING MAXIMUM│              │  INFORMATION USING MAXIMUM│
│       VALUE IN ROI 1      │              │       VALUE IN ROI 2      │
└──────────────────────────┘              └──────────────────────────┘
             │                                          │         S29
             ▼                                          ▼          │
┌──────────────────────────┐              ┌──────────────────────────┐
│    DETERMINE THRESHOLD    │              │    DETERMINE THRESHOLD    │
│  VALUE FOR BODY MOVEMENT  │── S26        │     VALUE FOR BODY        │
│   INFORMATION THAT MAY    │              │   MOVEMENT INFORMATION    │
│  CAUSE EXAMINATION TO BE  │              │   AROUND EXAMINATION SITE │
│        INTERRUPTED        │              └──────────────────────────┘
└──────────────────────────┘                          │
             │           S27          S31              ▼          S30
             ▼            │            │    ┌──────────────────────────┐
┌──────────────────────────┐  ┌────────────────────┐  │
│   OUTPUT CLASSIFICATION   │  │   OUTPUT BODY      │  │   OUTPUT CLASSIFICATION   │
│    RESULT AND ALERT       │  │ MOVEMENT WAVEFORM  │  │    RESULT AND ALERT       │
└──────────────────────────┘  └────────────────────┘  └──────────────────────────┘
             │                         │                          │
             ▼                         ▼                          ▼
          ( END )                   ( END )                    ( END )
```

FIG. 17

# FIG. 18

MAGNITUDE (AMPLITUDE)

124

TIME

SPACE

## FIG. 19A

## FIG. 19B

Europäisches
Patentamt

European
Patent Office

Office européen
des brevets

## EUROPEAN SEARCH REPORT

**Application Number**

EP 25 15 7179

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/378391 A1 (SUEHLING MICHAEL [DE] ET AL) 1 December 2022 (2022-12-01) * paragraphs [0003] - [0007], [0026] - [0033], [0040] - [0088]; claims 1, 2, 12, 16; figures 1-9 * | 1-5,7-15 | INV. G01R33/28 A61B5/11 A61B5/00 G01R33/565 |
| | ----- | | |
| X | US 2023/078113 A1 (YATSUO TAKESHI [JP] ET AL) 16 March 2023 (2023-03-16) * paragraphs [0004], [0009] - [0012], [0022] - [0026], [0041] - [0098]; claim 1; figures 1-8 * | 1-5,7-15 | |
| | ----- | | |
| X | US 2023/045497 A1 (SHOJI HIROKI [JP] ET AL) 9 February 2023 (2023-02-09) * paragraph [0012] - paragraph [0086]; claims 1-10; figures 1-4 * | 1-15 | |
| | ----- | | |

**TECHNICAL FIELDS
SEARCHED    (IPC)**

G01R
A61B

The present search report has been drawn up for all claims

| Place of search | Date of completion of the search | Examiner |
|---|---|---|
| Munich | 2 July 2025 | Faber-Jurk, Sonja |

EPO FORM 1503 03.82 (P04C01)

## ANNEX TO THE EUROPEAN SEARCH REPORT
## ON EUROPEAN PATENT APPLICATION NO.

EP 25 15 7179

This annex lists the patent family members relating to the patent documents cited in the above-mentioned European search report.
The members are as contained in the European Patent Office EDP file on
The European Patent Office is in no way liable for these particulars which are merely given for the purpose of information.

02-07-2025

| Patent document cited in search report | Publication date | Patent family member(s) | | Publication date |
|---|---|---|---|---|
| US 2022378391 A1 | 01-12-2022 | CN | 115474951 A | 16-12-2022 |
| | | EP | 4014875 A1 | 22-06-2022 |
| | | US | 2022378391 A1 | 01-12-2022 |
| US 2023078113 A1 | 16-03-2023 | CN | 115804584 A | 17-03-2023 |
| | | JP | 7489949 B2 | 24-05-2024 |
| | | JP | 2023043001 A | 28-03-2023 |
| | | US | 2023078113 A1 | 16-03-2023 |
| US 2023045497 A1 | 09-02-2023 | CN | 115919283 A | 07-04-2023 |
| | | JP | 7461913 B2 | 04-04-2024 |
| | | JP | 2023022669 A | 15-02-2023 |
| | | US | 2023045497 A1 | 09-02-2023 |
| | | US | 2024264258 A1 | 08-08-2024 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006346235 A